# DEMANDE DE BREVET EUROPEEN

(11) **EP 1 803 391 A1**
(43) Date de publication de la demande: **04.07.2007**
(21) Numéro de dépôt: 05113069.8
(22) Date de dépôt: 29.12.2005
(51) Int. Cl.: A61B 5/00, A61H 39/02, G09B 23/28

(54) **Outil de diagnostic**

(71) Demandeur: Martz, Alexandra, 1030 Bruxelles (BE)
(72) Inventeur: Martz, Alexandra, 1030 Bruxelles (BE)
(74) Mandataire: Quintelier, Claude

(57) **Abrégé**

Outil de diagnostic comprenant une planche représentant un corps humain ou animal, lequel corps est réparti en un ensemble de zones, un premier identificateur étant attribué à chaque zone, lequel outil comprend également par diagnostic à appliquer un ensemble d'indicateurs formé d'un premier indicateur reprenant le premier identificateur attribué à la première desdites zones où une personne chargée de faire le diagnostic doit appliquer au moins un doigt d'une de ses mains et une séquence de deuxièmes indicateurs reprenant les premiers identificateurs attribués aux points successifs attribués dans des zones ainsi identifiées où ladite personne doit successivement appliquer au moins un doigt de son autre main.

## Description

L'invention concerne un outil de diagnostic qui peut être utilisé pour diagnostiquer une maladie ou un dysfonctionnement du corps humain ou animal.

Un tel outil de diagnostic est généralement utilisé par des médecins, des kinésithérapeutes ou plus généralement des professionnels de la santé dans l'exercice de leur profession. Il permet de diagnostiquer une maladie ou un mauvais fonctionnement du corps humain ou animal, et ainsi au médecin de déterminer le traitement thérapeutique à appliquer.

Un désavantage des outils diagnostiques connus est qu'ils ne prennent en compte que des fractions du corps humain ou animal et non le fonctionnement du corps dans son ensemble.

L'invention a pour but de réaliser un outil de diagnostic permettant de mettre en relation les maladies et autres dysfonctionnements du corps humain ou animal ainsi que les symptômes qui lui sont associés avec des topographies de l'ensemble du corps.

A cette fin, un outil de diagnostic suivant l'invention est caractérisé en ce qu'il comprend une planche représentant un corps humain ou animal, lequel corps est réparti en un ensemble de zones, un premier identificateur étant attribué à chaque zone, lequel outil comprend également par diagnostic à appliquer un ensemble d'indicateurs formé d'un premier indicateur reprenant le premier identificateur attribué à la première desdites zones où une personne chargée de faire le diagnostic doit appliquer au moins un doigt d'une de ses mains et une séquence de deuxièmes indicateurs reprenant les premiers identificateurs attribués aux points successifs attribués dans des zones ainsi identifiées où ladite personne doit successivement appliquer au moins un doigt de son autre main.

Grâce au premier indicateur et à la séquence de deuxièmes indicateurs, la personne chargée de faire le diagnostic peut, par diagnostic à appliquer, parfaitement déterminer les endroits du corps où il ou elle doit d'abord appliquer au moins un doigt d'une main et ensuite un doigt de l'autre main. Ainsi en appliquant successivement des doigts en des endroits bien déterminés du corps, des couples de points sur le corps seront activés successivement, ce qui devrait engendrer une activation des systèmes nerveux, circulatoire et/ou osseux. Cette activation va alors permettre à la personne effectuant le diagnostic de constater comment le corps réagit et ceci permettra alors d'établir le diagnostic. Les différents premiers identificateurs donnent ainsi une topographie du corps, laquelle permet de mettre différents points de ce corps en relation l'un avec l'autre et d'agir sur l'ensemble du fonctionnement du corps.

Une première forme de réalisation préférentielle d'un outil de diagnostic suivant l'invention est caractérisée en ce que chacune desdites zones dudit ensemble de zones est subdivisée en un nombre prédéterminé de sous-zones, un deuxième identificateur étant attribué à chaque sous-zone, lesdits premiers et deuxièmes indicateurs reprenant également les deuxièmes identificateurs. La subdivision en sous-zones permet une topographie du corps qui est plus précise et permet également de réaliser le diagnostic de façon systématique.

Une deuxième forme de réalisation préférentielle d'un outil de diagnostic suivant l'invention est caractérisée en ce que lesdits premiers identificateurs comportent des numéros, lesquels sont attribués par ordre croissant en commençant par une partie inférieure dudit corps. L'usage de numéros par ordre croissant permet une approche logique et systématique de la répartition du corps.

L'invention sera maintenant décrite plus en détail à l'aide des dessins qui illustrent des exemples de réalisation d'un outil de diagnostic suivant l'invention.

Dans les dessins :
les fig. 1 a et b montrent un premier exemple d'un outil de diagnostic suivant l'invention et qui est destiné à diagnostiquer des allergies ; et
les fig. 2 a et b montrent un deuxième exemple d'un outil de diagnostic suivant l'invention et qui est destiné à diagnostiquer des problèmes de circulation sanguine.

Dans les dessins, une même référence a été attribuée à un même élément ou à un élément analogue.

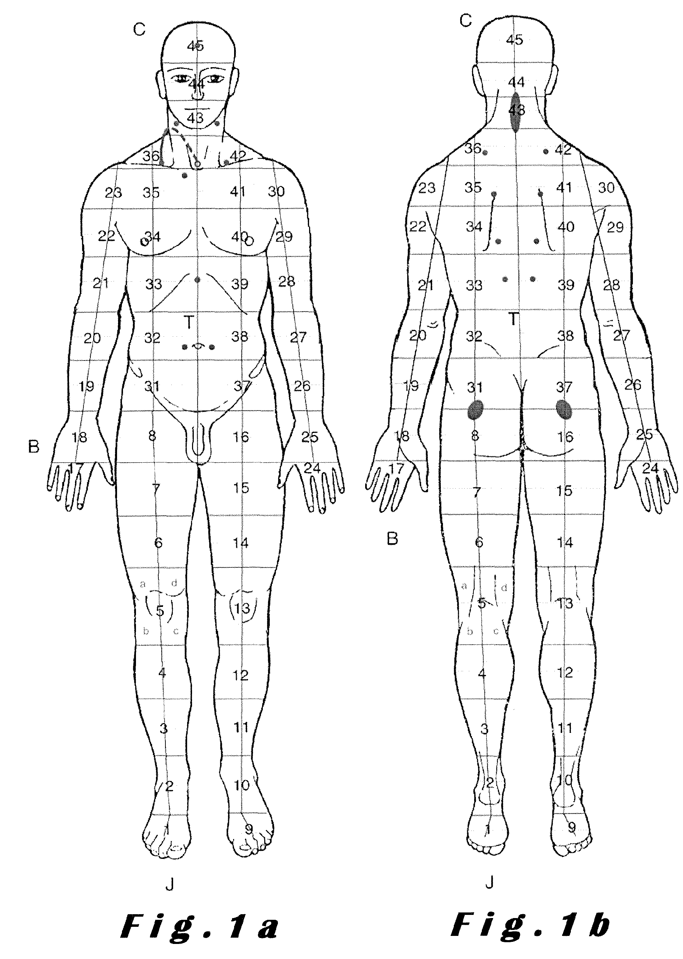

L'outil de diagnostic suivant l'invention et illustré à la fig. 1 comporte une planche représentant un corps humain, en l'occurrence la face ventrale (1a) et dorsale (1b) d'un homme. Bien entendu, l'invention n'est pas limitée à cette seule représentation de la face ventrale et dorsale d'un homme et peut également comprendre une représentation du profil droit ou gauche d'un homme ainsi que des représentations de la face dorsale, ventrale d'une femme et une représentation de ses profils. Puisque l'invention s'applique également à un outil de diagnostic pour le corps d'un animal, elle peut également comprendre des représentations du corps de différents animaux.

La planche est formée par une feuille de papier, de carton ou de matière synthétique ou naturelle sur laquelle la représentation du corps est imprimée. La planche peut également être formée par un écran d'ordinateur ou tout autre support permettant d'afficher des informations. Le corps est réparti en un ensemble de zones. Un premier identificateur est attribué à chaque zone. Dans l'exemple illustré à la fig. 1, les premiers identificateurs comportent des numéros attribués par ordre croissant en commençant par une partie inférieure du corps, en l'occurrence le pied droit. Il va de soi que d'autres formes des premiers identificateurs que des numéros peuvent être utilisés comme, par exemple, des lettres ou des symboles. Au lieu de débuter la numérotation par le pied, il est bien entendu également possible de la débuter en un autre endroit du corps comme, par exemple, une main ou la tête. II faut toutefois noter que la nomenclature n'indique le plus souvent qu'un hemi-corps et que les points représentés sur le support le sont de manière unilatérale.

Dans l'exemple illustré à la fig. 1, le corps est réparti en un ensemble de zones sensiblement rectangulaires. Il s'agit là bien entendu d'une forme de réalisation préférentielle, car la répartition en rectangles est simple et efficace à appliquer. Toutefois, d'autres répartitions que celles en rectangles peuvent également être réalisées, comme par exemple, des répartitions en triangles ou en trapèzes. La dimension de chaque zone peut soit être égale en surface, soit de surface différente. Il est toutefois préférable de tenir compte de la structure même du corps en appliquant la répartition en zones pour ainsi donner une structure cohérente à la zone. Ainsi, par exemple, le cerveau formera une seule zone.

Pour rendre le repérage des zones plus facile et plus rapide, les premiers identificateurs comprennent également des lettres majuscules. Ainsi, par exemple les lettres suivantes sont attribuées :
J : jambe
T : tronc
B : bras
C : crâne

Chaque zone de l'ensemble des zones est subdivisée en un nombre prédéterminé de sous-zones. Dans l'exemple illustré à la fig. 1, chaque zone rectangulaire est subdivisée en quatre rectangles afin d'affiner la répartition du corps de et rendre la détermination d'un point topographique sur le corps plus précise. A chaque sous-zone est attribué un deuxième identificateur. Dans l'exemple illustré à la fig. 1, les deuxièmes indicateurs sont formés par les lettres a, b, c et d attribuées chacune à une des sous-zones. L'attribution est similaire pour chaque sous-zone et suit le sens inverse des aiguilles d'une montre en commençant par le sous-rectangle supérieur gauche.

Pour chaque diagnostic à appliquer, l'outil de diagnostic suivant l'invention comprend également un ensemble d'indicateurs formé par un premier indicateur reprenant le premier identificateur attribué à la première desdites zones où une personne chargée de faire le diagnostic en question doit appliquer au moins un doigt d'une de ses mains. Ainsi, par exemple, dans la planche illustrée à la fig. 1 et le tableau 1, reprenant un diagnostic pour vérifier l'existence d'une allergie et la thérapie à appliquer, le premier indicateur est formé par T36c (repris sur la face ventrale)+ C43b+c repris sur la face dorsale du corps humain. Ceci signifie que la personne devant appliquer le diagnostic devra d'abord poser au moins un doigt d'une de ses mains, par exemple le pouce de sa main gauche, dans la zone T36, c'est-à-dire dans la zone 36 du tronc, plus particulièrement dans le sous rectangle (c) inférieur. En même temps, il ou elle appliquera au moins un autre doigt de sa même main dans la zone C43 de la face dorsale du crâne (comme indiqué dans la nomenclature), plus particulièrement dans le sous-rectangle (b) inférieur de gauche et (c) inférieur de droite. Afin d'encore faciliter la tâche de ladite personne, le premier indicateur est, de préférence, représenté par un symbole prédéterminé repris sur la représentation topographique illustrée dans les dessins. Ce symbole est par exemple formé par un rectangle ou une autre forme géométrique. L'ensemble d'indicateurs comporte également une séquence de deuxièmes indicateurs reprenant les premiers identificateurs attribués aux points successifs dans les zones ainsi identifiées où ladite personne doit successivement appliquer au moins un doigt de son autre main. Dans l'exemple des allergies illustré à la fig. 1, ce deuxième indicateur est formé par T33d +T39a et indique le point à appliquer par un des doigts de la main droite. Il est à noter que la séquence du deuxième indicateur doit être appliquée par la personne appliquant le diagnostic. Bien entendu ce deuxième indicateur peut également être identifié par une couleur dans la planche, de préférence la même que celle utilisée pour le premier indicateur.

En utilisant pour chaque diagnostic à appliquer une couleur différente, il est possible d'utiliser une même planche pour diagnostiquer plusieurs maladies différentes.

En utilisant l'ensemble d'indicateurs, la personne qui doit effectuer le diagnostic peut activer à chaque fois un couple de points sur le corps et ainsi engendrer une activation des systèmes osseux, circulatoire ou nerveux avec une prédominance de l'un ou de l'autre, voire une co-complémentarité. Ainsi, en appliquant d'abord au moins un doigt d'une main sur le point indiqué par le premier indicateur et ensuite au moins un doigt de l'autre main sur successivement chacun des points indiqués par le deuxième indicateur, ladite personne va induire une réaction physiologique dans le corps sur lequel le diagnostic doit s'appliquer. Cette réaction physiologique peut par exemple se traduire en un chargement électrique entre ces points et créer entre ces points un potentiel dans un champ électrique. Ainsi un courant électrique pourra circuler entre ces points. Ladite personne va ressentir ce courant dont les paramètres, comme par exemple l'intensité ou la polarité, auront changés et lui indiqueront la réaction du corps à diagnostiquer. Cette réaction fournira des informations nécessaires pour établir le diagnostic. Bien entendu cette réaction du corps sera totalement déterminée par le fonctionnement du corps. La réaction physiologique peut également se traduire en une réaction chimique dont les effets seront également ressentis par la personne qui effectue le diagnostic.

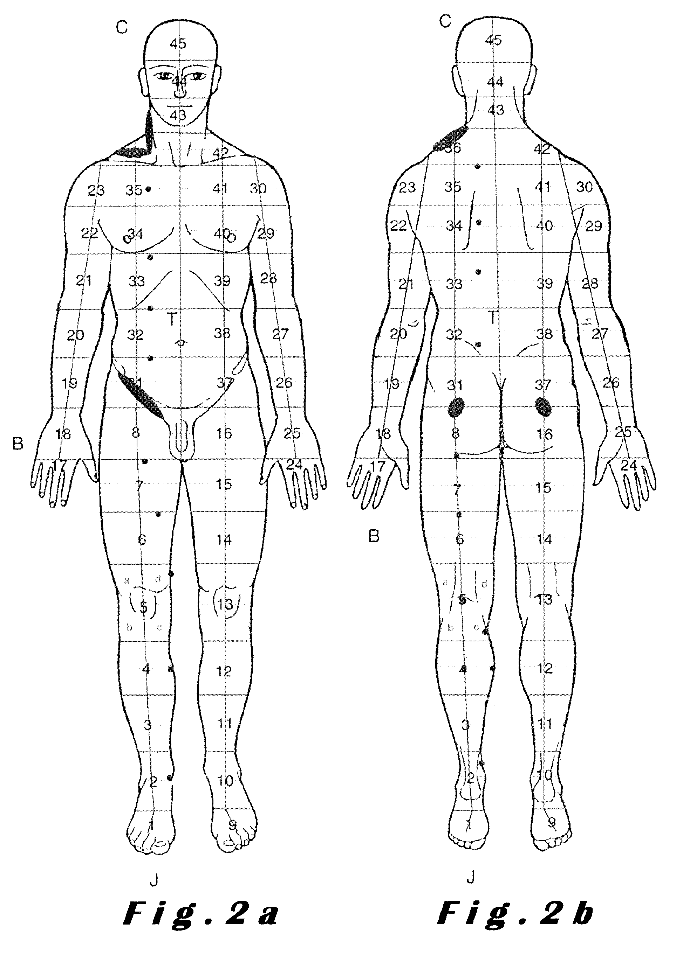

La planche illustrée à la fig. 2 ainsi que le tableau 2 reprennent un diagnostic pour vérifier l'existence d'un problème de circulation sanguine et la thérapie à appliquer. Dans ce diagnostic le premier indicateur est formé par le premier indicateur T 31 abc +J8d. Ceci signifie que la personne devant appliquer le diagnostic devra d'abord poser au moins un doigt d'une de ses mains, par exemple le majeur de sa main gauche, ou la paume de sa main dans la zone T31, c'est-à-dire dans la zone 31 du tronc, plus particulièrement dans les sous-rectangles a, b et c. En même temps il ou elle appliquera un autre doigt de cette même main dans la zone J8d de la jambe, plus particulièrement dans le sous-rectangle (d) supérieur de droite de la jambe, ou il veillera que la paume de sa main couvrira également cette dernière sous-zone.

Dans l'exemple des problèmes de circulation sanguine illustré à la fig. 2, le deuxième indicateur est formé par J2d, J4cd, J5d, J6d, J7d et indique les points à appliquer successivement par la main droite et ainsi de suite comme indiqué dans la nomenclature reprise ci-dessous.

L'outil de diagnostic suivant l'invention permet donc de mettre en relation les maladies ainsi que les symptômes qui lui sont associés avec des topographies précises du corps. L'outil permet non seulement de diagnostiquer mais également de traiter le corps, il a donc un aspect préventif et curatif. En effet lorsque les points indiqués par l'ensemble des indicateurs ont été activés, il sera également possible de stimuler le courant électrique ou la réaction chimique ainsi induit, ce qui va permettre au corps de se remettre à fonctionner dans son ensemble et de retrouver par ses propres moyens un équilibre qui aurait été perdu.

En agissant sur les os, le système circulatoire ou les nerfs, on agit de manière directe ou indirecte sur le système endocrinien, immunitaire, cardiaque, musculo-squelettique, lymphatique, cellulaire, tissulaire, physiologique ainsi que sur le système digestif, respiratoire, et neurovégétatif et donc de manière plus globale les différents systèmes constituants le corps humain. Le fait d'activer des courants électriques peut entraîner une dépolarisation membranaire entre les deux points stimulés. Cette dépolarisation membranaire est transmise au cerveau, qui activera alors les mécanismes intervenant dans le traitement du dysfonctionnement du corps. Les points activés peuvent également jouer un rôle de catalyseur chimique qui entraînera une activation enzymatique, donc plus généralement chimico-physiologique, qui, par effet de chaîne transmettra les informations nécessaires sur le rétablissement du système défaillant.

A titre d'illustration quelques exemples d'ensembles d'indicateurs seront repris ci-dessous. Dans les points diagnostics repris ci-dessous seul les points essentiels sont repris et ceci pour une raison de clarté.

### POINTS DIAGNOSTICS

### Appareil génital de la femme

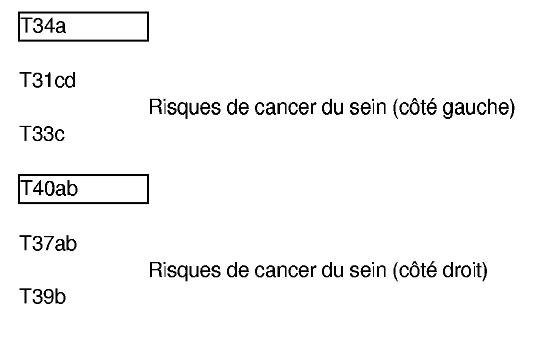

### Appareil génital de l'homme

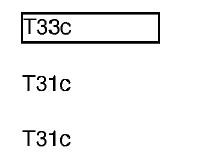

Problèmes de prostates, incontinance, troubles érectiles

### Digestion

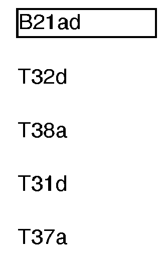

Problèmes de dos
Troubles digestifs : gonflements, douleurs...
Risque de cancer dans les cas graves (foie, pancréas)

### Allergies

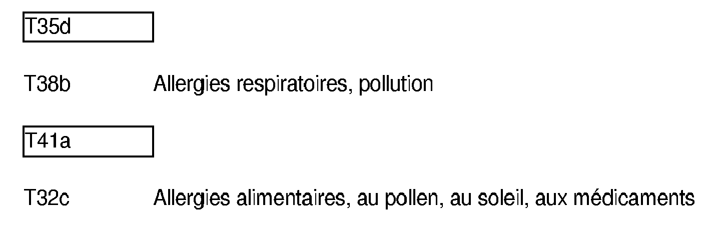

### Système circulatoire

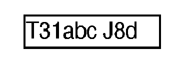

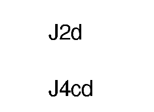

Problèmes circulatoires, sujet à phlébites

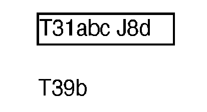

Risques cardiaques avec forte probabilité d'antécédents dans la famille

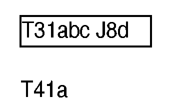

Risques d'AVC

### Fibromyalgie

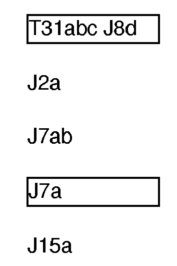

### Densité osseuse pour tous les os

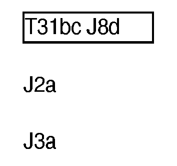

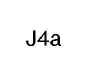

faible densité osseuse

### Infection

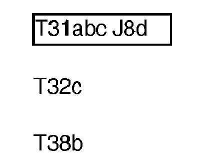

Infections dentaires, cutanées, piqûre d'insecte...

### Anxiété

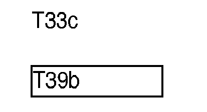

Patients sujets à angoisses, à crise d'anxiété, moral vascillant, phobies

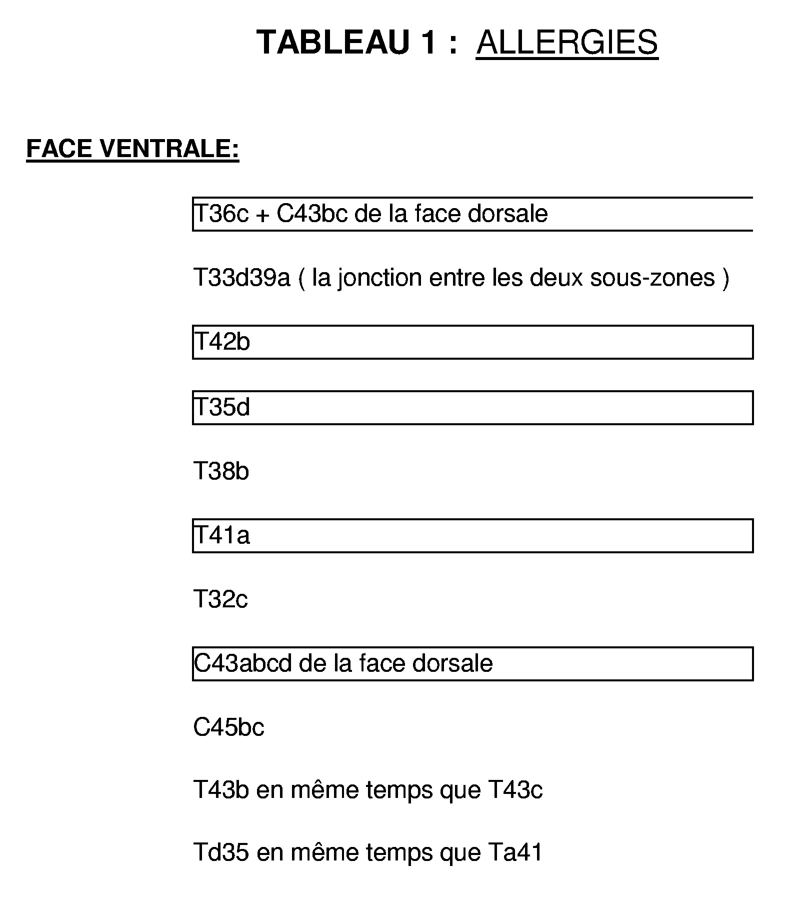

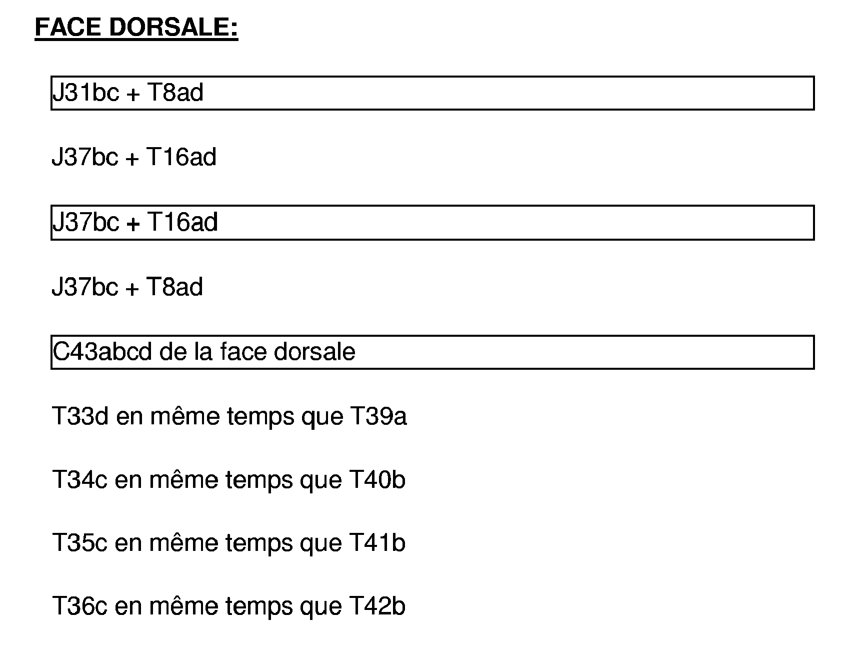

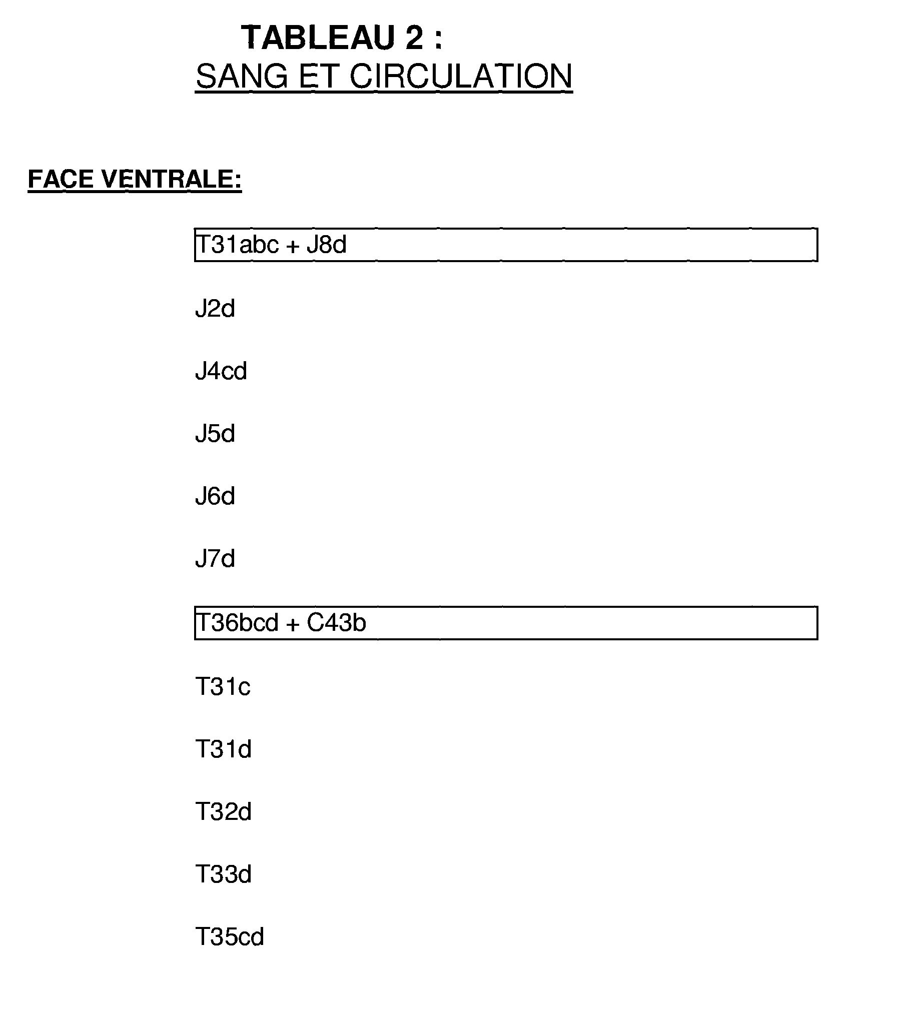

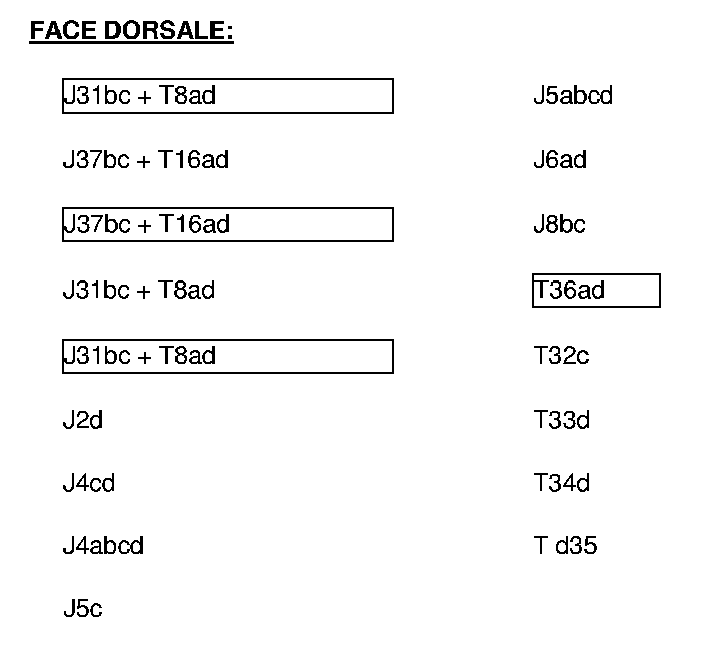

## Revendications

1. Outil de diagnostic **caractérisé en ce qu'**il comprend une planche représentant un corps humain ou animal, lequel corps est réparti en un ensemble de zones, un premier identificateur étant attribué à chaque zone, lequel outil comprend également par diagnostic à appliquer un ensemble d'indicateurs formé d'un premier indicateur reprenant le premier identificateur attribué à la première desdites zones où une personne chargée de faire le diagnostic doit appliquer au moins un doigt d'une de ses mains et une séquence de deuxièmes indicateurs reprenant les premiers identificateurs attribués aux points successifs attribués dans des zones ainsi identifiées où ladite personne doit successivement appliquer au moins un doigt de son autre main.

2. Outil de diagnostic suivant la revendication 1, **caractérisé en ce que** chacune desdites zones dudit ensemble de zones est subdivisée en un nombre prédéterminé de sous-zones, un deuxième identificateur étant attribué à chaque sous-zone, lesdits premiers et deuxièmes indicateurs reprenant également les deuxièmes identificateurs.

3. Outil de diagnostic suivant la revendication 1 ou 2, **caractérisé en ce que** le premier indicateur est attribué à la main gauche et les deuxièmes indicateurs à la main droite de ladite personne.

4. Outil de diagnostic suivant l'une des revendications 1 à 3, **caractérisé en ce que** lesdits premiers identificateurs comportent des numéros, lesquels sont attribués par ordre croissant en commençant par une partie inférieure dudit corps.
